# EUROPEAN PATENT SPECIFICATION

(11) **EP 1 357 941 B1**
(45) Date of publication and mention of the grant of the patent: **04.01.2006**
(21) Application number: 02711034.5
(22) Date of filing: 08.02.2002
(51) Int. Cl.: A61K 39/395, A61P 25/00, C07K 16/28

(54) **THERAPEUTICAL USE OF ANTI-MAG ANTIBODY FOR THE TREATMENT OF ISCHEMIC STROKE**
VERWENDUNG VON ANTIKÖRPER GEGEN MAG ZUR BEHANDLUNG ISCHÄMISCHER SCHLAGANFÄLLE
UTILISATION THERAPEUTIQUE D'ANTICORPS ANTI-MAG POUR TRAITER L'ACCIDENT ISCHEMIQUE

(30) Priority: 08.02.2001 GB 0103174
(43) Date of publication of application: 05.11.2003
(60) Divisional application: 05077130.2
(73) Proprietor: SMITHKLINE BEECHAM PLC, Brentford, Middlesex TW8 9GS (GB)
(72) Inventor: IRVING, Elaine Alison, Essex CM19 5AW (GB); VINSON, Mary, Essex CM19 5AW (GB)
(74) Representative: Learoyd, Stephanie Anne
(86) International application number: PCT/GB2002/000551
(87) International publication number: WO 2002/062383

(56) References cited:
- WO-A-00/63252
- WO-A-95/22344
- WO-A-99/25378
- WO-A-99/53945

## Description

### Field of the Invention

The present invention relates to a method of treatment of neurological diseases and to antibodies for use in such method.

### Background of the Invention

Stroke is a major cause of death and disability in the Western World. There is no approved therapy for the treatment of stroke other than t-PA which has to be administered within 3h of onset following a CT scan to exclude haemorrhage. To date most therapeutic agents directed towards the treatment of acute stroke (i.e. neuroprotection), have predominantly involved targeting glutamate receptors and their down stream signalling pathways known to be involved in acute cell death. However to date these strategies have proved unsuccessful in clinical trials and are often associated with dose-limiting side effects (Hill & Hachinski, The Lancet, 352 : (suppl III) 10-14 (1998)). Therefore there is a need for novel approaches directed towards the amelioration of cell death following the cessation of blood flow.

Following the onset of stroke, some degree of spontaneous functional recovery is observed in many patients, suggesting that the brain has the (albeit limited) ability to repair and/or remodel following injury. Agents that have the potential to enhance this recovery may therefore allow intervention to be made much later (potentially days) following the onset of cerebral ischaemia. Agents which are able to offer both acute neuroprotection and enhance functional recovery may provide significant advantages over current potential neuroprotective strategies.

The mechanisms underlying functional recovery are currently unknown. The sprouting of injured or non-injured axons has been proposed as one possible mechanism. However, although *in vivo* studies have shown that treatment of spinal cord injury or stroke with neurotrophic factors results in enhanced functional recovery and a degree of axonal sprouting, these do not prove a direct link between the degree of axonal sprouting and extent of functional recovery (Jakeman, et al. 1998, Exp. Neurol. 154 : 170-184, Kawamata et al. 1997, Proc Natl Acad. Sci. USA., 94:8179-8184, Ribotta, et al. 2000, J Neurosci. 20 : 5144-5152). Furthermore, axonal sprouting requires a viable neuron. In diseases such as stroke which is associated with extensive cell death, enhancement of functional recovery offered by a given agent post stroke may therefore be through mechanisms other than axonal sprouting such as differentiation of endogenous stem cells, activation of redundant pathways, changes in receptor distribution or excitability of neurons or glia (Fawcett & Asher, 1999, Brain Res. Bulletin, 49 : 377-391, Homer & Gage, 2000, Nature 407 963-970).

The limited ability of the central nervous system (CNS) to repair following injury is thought in part to be due to molecules within the CNS environment that have an inhibitory effect on axonal sprouting (neurite outgrowth). CNS myelin is thought to contain inhibitory molecules (Schwab ME and Caroni P (1988) *J. Neurosci.* **8,** 2381-2193). Two myelin proteins, myelin-associated glycoprotein (MAG) and Nogo have been cloned and identified as putative inhibitors of neurite outgrowth (Sato S. et al (1989) *Biochem. Biophys. Res. Comm*.**163**,1473-1480; McKerracher L et al (1994) *Neuron* **13**, 805-811; Mukhopadhyay G et al (1994) *Neuron* 13, 757-767; Torigoe K and Lundborg G (1997) *Exp. Neurology* **150**, 254-262; Schafer et al (1996) *Neuron* 16, 1107-1113; WO9522344; W09701352; Prinjha R et al (2000) *Nature* **403**, 383-384; Chen MS et al (2000) *Nature* **403**, 434-439; GrandPre T et al (2000) *Nature* **403**, 439-444; US005250414A; WO200005364A1; WO0031235).

Myelin-associated glycoprotein is a cell surface transmembrane molecule expressed on the surface of myelin consisting of five extracellular immunoglobulin domains, a single transmembrane domain and an intracellular domain. MAG expression is restricted to myelinating glia in the CNS and peripheral nervous system (PNS). MAG is thought to interact with neuronal receptor(s) which mediate effects on the neuronal cytoskeleton including neurofilament phosphorylation and inhibition of neurite outgrowth *in vitro.* Although antagonists of MAG have been postulated as useful for the promotion of axonal sprouting following injury (WO9522344, WO9701352 and WO9707810), these claims are not supported by *in vivo* data. Furthermore, the role of MAG as an inhibitor of axonal sprouting from CNS neurons *in vivo* is not proven (Li CM et al (1994) *Nature* **369**, 747-750; Montag, D et al (1994) *Neuron* 13, 229-246; Lassmann H et al (1997) *GLIA* **19**,104-110; Li C et al (1998) *J. Neuro. Res.* **51**,210-217; Yin X et al (1998) *J. Neurosci.* **18**, 1953-1962; Bartsch U et al (1995) *Neuron* **15** 1375-1381; Li M et al (1996) **46**,404-414).

It has now been found that an anti-MAG monoclonal antibody, when administered either directly into the brain or intravenously following focal cerebral ischaemia in the rat (a model of stroke), provides neuroprotection and enhances functional recovery. Therefore anti-MAG antibodies or MAG antagonists provide potential therapeutic agents for both acute neuroprotection as well as the promotion of functional recovery following stroke. Other agents that disrupt the interation between MAG and it's neuronal receptors (such as receptor antagonists or antibodies recognising the MAG receptor(s)) may also provide this therapeutic benefit.

The process of neurodegeneration underlies many neurological diseases including acute diseases such as stroke, traumatic brain injury and spinal cord injury as well as chronic diseases including Alzheimer's disease, fronto-temporal dementias (tauopathies), peripheral neuropathy, Parkinson's disease, Huntington's disease and multiple sclerosis. Anti-MAG mabs or MAG antagonists therefore may be useful in the treatment of these diseases, by both ameliorating the cell death associated with these disorders and promoting functional recovery.

### Brief Summary of the Invention

In one aspect, the invention provides the use of an anti-MAG antibody, in the preparation of an intravenously administrable medicament for treatment or prophylaxis of ischaemic stroke.

In another aspect, the invention provides the use of a anti-MAG antibody, in the preparation of an intravenously administrable medicament for inhibiting neurodegeneration and/or promoting functional recovery in a human patient suffering from, or at risk of developing, an ischaemic stroke.

In yet another aspect, the present invention provides an intravenously administrable pharmaceutical composition which comprises one (or more) anti-MAG antibodies and a pharmaceutically acceptable carrier.

Other aspects and advantages of the present invention are described further in the detailed description and the preferred embodiments thereof.

### Anti-MAG Antibody

The antibody is preferably raised to human MAG and is preferably a monoclonal antibody (mAb), which is preferably humanised or reshaped.

In a preferred aspect the mAb is class IgG.

In a further preferred aspect the antibody is a neutralising antibody.

"Neutralising" refers to substantial inhibition of MAG function including its binding to neurones and inhibition of neurite outgrowth.

"Substantial inhibition" refers to 75%, more preferably 85%, most preferably 95% inhibition measured in *in vitro* tests.

"Altered antibody" refers to a protein encoded by an altered immunoglobulin coding region, which may be obtained by expression in a selected host cell. Such altered antibodies include engineered antibodies (e.g., chimeric, reshaped, humanized or vectored antibodies) or antibody fragments lacking all or part of an immunoglobulin constant region, e.g., Fv, Fab, or F(ab)₂ and the like.

"Altered immunoglobulin coding region" refers to a nucleic acid sequence encoding altered antibody. When the altered antibody is a CDR-grafted or humanized antibody, the sequences that encode the complementarity determining regions (CDRs) from a non-human immunoglobulin are inserted into a first immunoglobulin partner comprising human variable framework sequences. Optionally, the first immunoglobulin partner is operatively linked to a second immunoglobulin partner.

"First immunoglobulin partner" refers to a nucleic acid sequence encoding a human framework or human immunoglobulin variable region in which the native (or naturally-occurring) CDR-encoding regions are replaced by the CDR-encoding regions of a donor antibody. The human variable region can be an immunoglobulin heavy chain, a light chain (or both chains), an analog or functional fragments thereof. Such CDR regions, located within the variable region of antibodies (immunoglobulins) can be determined by known methods in the art. For example Kabat et al. (Sequences of Proteins of Immunological Interest, 4th Ed., U.S. Department of Health and Human Services, National Institutes of Health (1987)) disclose rules for locating CDRs. In addition, computer programs are known which are useful for identifying CDR regions/structures.

"Second immunoglobulin partner" refers to another nucleotide sequence encoding a protein or peptide to which the first immunoglobulin partner is fused in frame or by means of an optional conventional linker sequence (i.e., operatively linked). Preferably it is an immunoglobulin gene. The second immunoglobulin partner may include a nucleic acid sequence encoding the entire constant region for the same (i.e., homologous - the first and second altered antibodies are derived from the same source) or an additional (i.e., heterologous) antibody of interest. It may be an immunoglobulin heavy chain or light chain (or both chains as part of a single polypeptide). The second immunoglobulin partner is not limited to a particular immunoglobulin class or isotype. In addition, the second immunoglobulin partner may comprise part of an immunoglobulin constant region, such as found in a Fab, or F(ab)₂ (i.e., a discrete part of an appropriate human constant region or framework region). Such second immunoglobulin partner may also comprise a sequence encoding an integral membrane protein exposed on the outer surface of a host cell, e.g., as part of a phage display library, or a sequence encoding a protein for analytical or diagnostic detection, e.g., horseradish peroxidase, β-galactosidase, etc.

The terms Fv, Fc, Fd, Fab, or F(ab)₂ are used with their standard meanings (see, e.g., Harlow et al., Antibodies A Laboratory Manual, Cold Spring Harbor Laboratory, (1988)).

As used herein, an "engineered antibody" describes a type of altered antibody, i.e., a full-length synthetic antibody (e.g., a chimeric, reshaped or humanized antibody as opposed to an antibody fragment) in which a portion of the light and/or heavy chain variable domains of a selected acceptor antibody are replaced by analogous parts from one or more donor antibodies which have specificity for the selected epitope. For example, such molecules may include antibodies characterized by a humanized heavy chain associated with an unmodified light chain (or chimeric light chain), or vice versa. Engineered antibodies may also be characterized by alteration of the nucleic acid sequences encoding the acceptor antibody light and/or heavy variable domain framework regions in order to retain donor antibody binding specificity. These antibodies can comprise replacement of one or more CDRs (preferably all) from the acceptor antibody with CDRs from a donor antibody described herein.

A "chimeric antibody" refers to a type of engineered antibody which contains a naturally-occurring variable region (light chain and heavy chains) derived from a donor antibody in association with light and heavy chain constant regions derived from an acceptor antibody.

A "humanized antibody" refers to a type of engineered antibody having its CDRs derived from a non-human donor immunoglobulin, the remaining immunoglobulin-derived parts of the molecule being derived from one (or more) human immunoglobulin(s). In addition, framework support residues may be altered to preserve binding affinity (see, e.g., Queen et al., Proc. Natl Acad Sci USA, 86:10029-10032 (1989), Hodgson et al., Bio/Technology, 9:421 (1991)).

"Reshaped human antibody" refers to an altered antibody in which minimally at least one CDR from a first human monoclonal donor antibody is substituted for a CDR in a second human acceptor antibody. Preferrably all six CDRs are replaced. More preferrably an entire antigen combining region (e.g., Fv, Fab or F(ab')₂) from a first human donor monoclonal antibody is substituted for the corresponding region in a second human acceptor monoclonal antibody. Most preferrably the Fab region from a first human donor is operatively linked to the appropriate constant regions of a second human acceptor antibody to form a full length monoclonal antibody.

A "vectored antibody" refers to an antibody to which an agent has been attached to improve transport through the blood brain barrier (BBB). The attachment may be chemical or alternatively the moeity can be engineered into the antibody. One example is to make a chimera with an antibody directed towards a brain capilliary endothelial cell receptor eg an anti-insulin receptor antibody or anti-transferrin receptor antibody (Saito et al (1995) *Proc. Natl. Acad. Sci. USA* **92** 10227-31; Pardridge et al (1995) *Pharm. Res.* **12** 807-816; Broadwell et al (1996) *Exp. Neurol*. **142** 47-65; Bickel et al (1993) *Proc Natl. Acad Sci. USA* **90**, 2618-2622; Friden et al (1996) *J. Pharm. Exp. Ther.* **278** 1491-1498, US5182107, US5154924, US5833988, US5527527). Once bound to the receptor, both components of the bispecific antibody pass across the BBB by the process of transcytosis. Alternatively the agent may be a ligand which binds such cell surface receptors eg insulin, transferrin or low density lipoprotein (Descamps et al (1996) *Am. J. Physiol.* **270** H1149-H1158; Duffy et al (1987) *Brain Res.* **420** 32-38; Dehouck et al (1997) *J. Cell Biol.* **1997** 877-889). Naturally occuring peptides such as penetratin and SynB1 and Syn B3 which are known to improve transport across the BBB can also be used (Rouselle et al (2000) *Mol. Pharm.***57***,* 679-686 and Rouselle et al (2001) *Journal of Pharmacology and Experimental Therapeutics* **296**,124-131).

The term "donor antibody" refers to an antibody (monoclonal, or recombinant) which contributes the nucleic acid sequences of its variable regions, CDRs, or other functional fragments or analogs thereof to a first immunoglobulin partner, so as to provide the altered immunoglobulin coding region and resulting expressed altered antibody with the antigenic specificity and neutralizing activity characteristic of the donor antibody.

The term "acceptor antibody" refers to an antibody (monoclonal, or recombinant) heterologous to the donor antibody, which contributes all (or any portion, but preferably all) of the nucleic acid sequences encoding its heavy and/or light chain framework regions and/or its heavy and/or light chain constant regions to the first immunoglobulin partner. Preferably a human antibody is the acceptor antibody.
"CDRs" are defined as the complementarity determining region amino acid sequences of an antibody which are the hypervariable regions of immunoglobulin heavy and light chains. See, e.g., Kabat et al., Sequences of Proteins of Immunological Interest, 4th Ed., U.S. Department of Health and Human Services, National Institutes of Health (1987). There are three heavy chain and three light chain CDRs (or CDR regions) in the variable portion of an immunoglobulin. Thus, "CDRs" as used herein refers to all three heavy chain CDRs, or all three light chain CDRs (or both all heavy and all light chain CDRs, if appropriate). ). The structure and protein folding of the antibody may mean that other residues are considered part of the antigen binding region and would be understood to be so by a skilled person. See for example Chothia et al., (1989) Conformations of immunoglobulin hypervariable regions; Nature 342, p877-883.

CDRs provide the majority of contact residues for the binding of the antibody to the antigen or epitope. CDRs of interest in this invention are derived from donor antibody variable heavy and light chain sequences, and include analogs of the naturally occurring CDRs, which analogs also share or retain the same antigen binding specificity and/or neutralizing ability as the donor antibody from which they were derived.

A "functional fragment" is a partial heavy or light chain variable sequence (e.g., minor deletions at the amino or carboxy terminus of the immunoglobulin variable region) which retains the same antigen binding specificity and/or neutralizing ability as the antibody from which the fragment was derived.

An "analog" is an amino acid sequence modified by at least one amino acid, wherein said modification can be chemical or a substitution or a rearrangement of a few amino acids (i.e., no more than 10), which modification permits the amino acid sequence to retain the biological characteristics, e.g., antigen specificity and high affinity, of the unmodified sequence. For example, (silent) mutations can be constructed, via substitutions, when certain endonuclease restriction sites are created within or surrounding CDR-encoding regions.. The present invention contemplates the use of analogs of the antibody of the invention. It is well known that minor changes in amino acid or nucleic acid sequences may lead eg to an allelic form of the original protein which retains substantially similar properties. Thus analogs of the antibody of the invention includes those in which the CDRs in the hypervariable region of the heavy and light chains are at least 80% homologous, preferably at least 90 % homologous and more preferably at least 95 % homologous to the CDRs as defined above as CDRH1, CDRH2, CDRH3, CDRL1, CDRL2 and CDRL3 and retain MAG neutralising activity. Amino acid sequences are are at least 80% homologous if they have 80% identical amino acid residues in a like position when the sequences are aligned optimally, gaps or insertions being counted as non-identical residues.

Analogs may also arise as allelic variations. An "allelic variation or modification" is an alteration in the nucleic acid sequence. Such variations or modifications may be due to degeneracy in the genetic code or may be deliberately engineered to provide desired characteristics. These variations or modifications may or may not result in alterations in any encoded amino acid sequence.

The term "effector agents" refers to non-protein carrier molecules to which the altered antibodies, and/or natural or synthetic light or heavy chains of the donor antibody or other fragments of the donor antibody may be associated by conventional means. Such non-protein carriers can include conventional carriers used in the diagnostic field, e.g., polystyrene or other plastic beads, polysaccharides, e.g., as used in the BIAcore [Pharmacia] system, or other non-protein substances useful in the medical field and safe for administration to humans and animals. Other effector agents may include a macrocycle, for chelating a heavy metal atom, or radioisotopes. Such effector agents may also be useful to increase the half-life of the altered antibodies, e.g., polyethylene glycol.

A neutralising antibody specific for MAG has been described (Poltorak et al (1987) *Journal of Cell Biology* **105**,1893-1899, DeBellard et al (1996) *Mol. Cell. Neurosci.* **7**, 89-101; Tang et al (1997) *Mol. Cell. Neurosci.* **9**, 333-346; Torigoe K and Lundborg G (1997) *Exp. Neurology* **150**, 254-262) and is commercially available (MAB1567 (Chemicon)).

A further aspect of the invention provides a pharmaceutical composition comprising an anti-MAG antibody together with a pharmaceutically acceptable diluent or carrier.
The altered antibody is preferably monoclonal, preferably humanised.

Alternatively, one can construct antibodies, altered antibodies and fragments, by immunizing a non-human species (for example, bovine, ovine, monkey, chicken, rodent (e.g., murine and rat), etc.) to generate a desirable immunoglobulin upon presentation with native MAG from any species against which antibodies cross reactive with human MAG can be generated, eg human or chicken.. Conventional hybridoma techniques are employed to provide a hybridoma cell line secreting a non-human mAb to MAG. Such hybridomas are then screened for binding using MAG coated to 384- or 96-well plates, with biotinylated MAG bound to a streptavidin coated plate. or in a homogenous europium-APC linked immunoassay using biotinylated MAG.

A native human antibody can be produced in a human antibody mouse such as the "Xenomouse" (Abgenix) where the mouse immunoglobulin genes have been removed and genes encoding the human immunoglobulins have been inserted into the mouse chromosome. The mice are immunised as normal and develop an antibody reponse that is derived from the human genes. Thus the mouse produces human antibodies obviating the need to humanize the after selection of positive hybridomas. (See Green L.L., *J Immunol Methods* 1999 Dec 10;231(1-2):11-23)

A Fab fragment contains the entire light chain and amino terminal portion of the heavy chain; and an F(ab')₂ fragment is the fragment formed by two Fab fragments bound by disulfide bonds. Fab fragments and F(ab')₂ fragments can be obtained by conventional means, e.g., cleavage of mAb with the appropriate proteolytic enzymes, papain and/or pepsin, or by recombinant methods. The Fab and F(ab')₂ fragments are useful themselves as therapeutic or prophylactic, and as donors of sequences including the variable regions and CDR sequences useful in the formation of recombinant or humanized antibodies as described herein.

The Fab and F(ab')₂ fragments can also be constructed via a combinatorial phage library (see, e.g., Winter et al., Ann. Rev. Immunol., 12:433-455 (1994)) or via immunoglobulin chain shuffling (see, e.g., Marks et al., Bio/Technology, 10:779-783 (1992), which are both hereby incorporated by reference in their entirety.

Thus human antibody fragments (Fv, scFv, Fab) specific for MAG can be isolated using human antibody fragment phage display libraries. A library of bacteriophage particles, which display the human antibody fragment proteins, are panned against the MAG protein. Those phage displaying antibody fragments that bind the MAG are retained from the library and clonally amplified. The human antibody genes are then exicised from the specific bacteriophage and inserted into human IgG expression constructs containing the human IgG constant regions to form the intact human IgG molecule with the variable regions from the isolated bacteriophage specific for MAG.

The donor antibodies may contribute sequences, such as variable heavy and/or light chain peptide sequences, framework sequences, CDR sequences, functional fragments, and analogs thereof, and the nucleic acid sequences encoding them, useful in designing and obtaining various altered antibodies which are characterized by the antigen binding specificity of the donor antibody.

Taking into account the degeneracy of the genetic code, various coding sequences may be constructed which encode the variable heavy and light chain amino acid sequences, and CDR sequences as well as functional fragments and analogs thereof which share the antigen specificity of the donor antibody. Isolated nucleic acid sequences, or fragments thereof, encoding the variable chain peptide sequences or CDRs can be used to produce altered antibodies, e.g., chimeric or humanized antibodies, or other engineered antibodies when operatively combined with a second immunoglobulin partner.

Altered immunoglobulin molecules can encode altered antibodies which include engineered antibodies such as chimeric antibodies and humanized antibodies. A desired altered immunoglobulin coding region contains CDR-encoding regions that encode peptides having the antigen specificity of an anti-MAG antibody, preferably a high affinity antibody, inserted into a first immunoglobulin partner (a human framework or human immunoglobulin variable region).

Preferably, the first immunoglobulin partner is operatively linked to a second immunoglobulin partner. The second immunoglobulin partner is defined above, and may include a sequence encoding a second antibody region of interest, for example an Fc region. Second immunoglobulin partners may also include sequences encoding another immunoglobulin to which the light or heavy chain constant region is fused in frame or by means of a linker sequence. Engineered antibodies directed against functional fragments or analogs of MAG may be designed to elicit enhanced binding.

The second immunoglobulin partner may also be associated with effector agents as defined above, including non-protein carrier molecules, to which the second immunoglobulin partner may be operatively linked by conventional means.

Fusion or linkage between the second immunoglobulin partners, e.g., antibody sequences, and the effector agent may be by any suitable means, e.g., by conventional covalent or ionic bonds, protein fusions, or hetero-bifunctional cross-linkers, e.g., carbodiimide, glutaraldehyde, and the like. Such techniques are known in the art and readily described in conventional chemistry and biochemistry texts.

Additionally, conventional linker sequences which simply provide for a desired amount of space between the second immunoglobulin partner and the effector agent may also be constructed into the altered immunoglobulin coding region. The design of such linkers is well known to those of skill in the art.

In still a further embodiment, the antibody of the invention may have attached to it an additional agent. For example, the procedure of recombinant DNA technology may be used to produce an engineered antibody of the invention in which the Fc fragment or CH2-CH3 domain of a complete antibody molecule has been replaced by an enzyme or other detectable molecule (i.e., a polypeptide effector or reporter molecule).

The second immunoglobulin partner may also be operatively linked to a non-immunoglobulin peptide, protein or fragment thereof heterologous to the CDR-containing sequence having the antigen specificity of anti-MAG antibody. The resulting protein may exhibit both anti-MAG antigen specificity and characteristics of the non-immunoglobulin upon expression. That fusion partner characteristic may be, e.g., a functional characteristic such as another binding or receptor domain, or a therapeutic characteristic if the fusion partner is itself a therapeutic protein, or additional antigenic characteristics.

Another desirable protein of this invention may comprise a complete antibody molecule, having full length heavy and light chains, or any discrete fragment thereof, such as the Fab or F(ab')₂ fragments, a heavy chain dimer, or any minimal recombinant fragments thereof such as an Fᵥ or a single-chain antibody (SCA) or any other molecule with the same specificity as the selected donor mAb. Such protein may be used in the form of an altered antibody, or may be used in its unfused form.

Whenever the second immunoglobulin partner is derived from an antibody different from the donor antibody, e.g., any isotype or class of immunoglobulin framework or constant regions, an engineered antibody results. Engineered antibodies can comprise immunoglobulin (Ig) constant regions and variable framework regions from one source, e.g., the acceptor antibody, and one or more (preferably all) CDRs from the donor antibody. In addition, alterations, e.g., deletions, substitutions, or additions, of the acceptor mAb light and/or heavy variable domain framework region at the nucleic acid or amino acid levels, or the donor CDR regions may be made in order to retain donor antibody antigen binding specificity.

Such engineered antibodies are designed to employ one (or both) of the variable heavy and/or light chains of the anti-MAG mAb or one or more of the heavy or light chain CDRs. The engineered antibodies may be neutralising, as above defined.

Such engineered antibodies may include a humanized antibody containing the framework regions of a selected human immunoglobulin or subtype, or a chimeric antibody containing the human heavy and light chain constant regions fused to the anti-MAG antibody functional fragments. A suitable human (or other animal) acceptor antibody may be one selected from a conventional database, e.g., the KABAT® database, Los Alamos database, and Swiss Protein database, by homology to the nucleotide and amino acid sequences of the donor antibody. A human antibody characterized by a homology to the framework regions of the donor antibody (on an amino acid basis) may be suitable to provide a heavy chain constant region and/or a heavy chain variable framework region for insertion of the donor CDRs. A suitable acceptor antibody capable of donating light chain constant or variable framework regions may be selected in a similar manner. It should be noted that the acceptor antibody heavy and light chains are not required to originate from the same acceptor antibody.

Desirably the heterologous framework and constant regions are selected from human immunoglobulin classes and isotypes, such as IgG (subtypes 1 through 4), IgM, IgA, and IgE. However, the acceptor antibody need not comprise only human immunoglobulin protein sequences. For instance a gene may be constructed in which a DNA sequence encoding part of a human immunoglobulin chain is fused to a DNA sequence encoding a non-immunoglobulin amino acid sequence such as a polypeptide effector or reporter molecule.

Preferably, in a humanized antibody, the variable domains in both human heavy and light chains have been engineered by one or more CDR replacements. It is possible to use all six CDRs, or various combinations of less than the six CDRs. Preferably all six CDRs are replaced. It is possible to replace the CDRs only in the human heavy chain, using as light chain the unmodified light chain from the human acceptor antibody. Alternatively, a compatible light chain may be selected from another human antibody by recourse to the conventional antibody databases. The remainder of the engineered antibody may be derived from any suitable acceptor human immunoglobulin.

The engineered humanized antibody thus preferably has the structure of a natural human antibody or a fragment thereof, and possesses the combination of properties required for effective therapeutic use.

It will be understood by those skilled in the art that an engineered antibody may be further modified by changes in variable domain amino acids without necessarily affecting the specificity and high affinity of the donor antibody (i.e., an analog). It is anticipated that heavy and light chain amino acids may be substituted by other amino acids either in the variable domain frameworks or CDRs or both.

In addition, the constant region may be altered to enhance or decrease selective properties of the molecules of the instant invention. For example, dimerization, binding to Fc receptors, or the ability to bind and activate complement (see, e.g., Angal et al., Mol. Immunol, 30:105-108 (1993), Xu et al., J. Biol. Chem, 269:3469-3474 (1994), Winter et al., EP 307,434-B).

An altered antibody which is a chimeric antibody differs from the humanized antibodies described above by providing the entire non-human donor antibody heavy chain and light chain variable regions, including framework regions, in association with human immunoglobulin constant regions for both chains.

Preferably, the variable light and/or heavy chain sequences and the CDRs of suitable donor mAbs, and their encoding nucleic acid sequences, are utilized in the construction of altered antibodies, preferably humanized antibodies, of this invention, by the following process. The same or similar techniques may also be employed to generate other embodiments of this invention.

A hybridoma producing a selected donor mAb is conventionally cloned, and the DNA of its heavy and light chain variable regions obtained by techniques known to one of skill in the art, e.g., the techniques described in Sambrook *et al*., (Molecular Cloning (A Laboratory Manual), 2nd edition, Cold Spring Harbor Laboratory (1989)). The variable heavy and light regions containing at least the CDR-encoding regions and those portions of the acceptor mAb light and/or heavy variable domain framework regions required in order to retain donor mAb binding specificity, as well as the remaining immunoglobulin-derived parts of the antibody chain derived from a human immunoglobulin are obtained using polynucleotide primers and reverse transcriptase. The CDR-encoding regions are identified using a known database and by comparison to other antibodies.

A mouse/human chimeric antibody may then be prepared and assayed for binding ability. Such a chimeric antibody contains the entire non-human donor antibody V_{H} and V_{L} regions, in association with human Ig constant regions for both chains.

Homologous framework regions of a heavy chain variable region from a human antibody may be identified using computerized databases, e.g., KABAT®, and a human antibody having homology to the donor antibody will be selected as the acceptor antibody. A suitable light chain variable framework region can be designed in a similar manner.

A humanized antibody may be derived from the chimeric antibody, or preferably, made synthetically by inserting the donor mAb CDR-encoding regions from the heavy and light chains appropriately within the selected heavy and light chain framework. Alternatively, a humanized antibody can be made using standard mutagenesis techniques. Thus, the resulting humanized antibody contains human framework regions and donor mAb CDR-encoding regions. There may be subsequent manipulation of framework residues. The resulting humanized antibody can be expressed in recombinant host cells, e.g., COS, CHO or myeloma cells.

A conventional expression vector or recombinant plasmid is produced by placing these coding sequences for the antibody in operative association with conventional regulatory control sequences capable of controlling the replication and expression in, and/or secretion from, a host cell. Regulatory sequences include promoter sequences, e.g., CMV promoter, and signal sequences, which can be derived from other known antibodies. Similarly, a second expression vector can be produced having a DNA sequence which encodes a complementary antibody light or heavy chain. Preferably this second expression vector is identical to the first except insofar as the coding sequences and selectable markers are concerned, so to ensure as far as possible that each polypeptide chain is fimctionally expressed. Alternatively, the heavy and light chain coding sequences for the altered antibody may reside on a single vector.

A selected host cell is co-transfected by conventional techniques with both the first and second vectors (or simply transfected by a single vector) to create the transfected host cell of the invention comprising both the recombinant or synthetic light and heavy chains. The transfected cell is then cultured by conventional techniques to produce the engineered antibody of the invention. The humanized antibody which includes the association of both the recombinant heavy chain and/or light chain is screened from culture by appropriate assay, such as ELISA or RIA. Similar conventional techniques may be employed to construct other altered antibodies and molecules.

Suitable vectors for the cloning and subcloning steps employed in the methods and construction of the compositions of this invention may be selected by one of skill in the art. For example, the conventional pUC series of cloning vectors, may be used. One vector, pUC19, is commercially available from supply houses, such as Amersham (Buckinghamshire, United Kingdom) or Pharmacia (Uppsala, Sweden). Additionally, any vector which is capable of replicating readily, has an abundance of cloning sites and selectable genes (e.g., antibiotic resistance), and is easily manipulated may be used for cloning. Thus, the selection of the cloning vector is not a limiting factor in this invention.

Similarly, the vectors employed for expression of the antibodies may be selected by one of skill in the art from any conventional vector. The vectors also contain selected regulatory sequences (such as CMV promoters) which direct the replication and expression of heterologous DNA sequences in selected host cells. These vectors contain the above described DNA sequences which code for the antibody or altered immunoglobulin coding region. In addition, the vectors may incorporate the selected immunoglobulin sequences modified by the insertion of desirable restriction sites for ready manipulation.

The expression vectors may also be characterized by genes suitable for amplifying expression of the heterologous DNA sequences, e.g., the mammalian dihydrofolate reductase gene (DHFR). Other preferable vector sequences include a poly A signal sequence, such as from bovine growth hormone (BGH) and the betaglobin promoter sequence (betaglopro). The expression vectors useful herein may be synthesized by techniques well known to those skilled in this art.

The components of such vectors, e.g. replicons, selection genes, enhancers, promoters, signal sequences and the like, may be obtained from commercial or natural sources or synthesized by known procedures for use in directing the expression and/or secretion of the product of the recombinant DNA in a selected host. Other appropriate expression vectors of which numerous types are known in the art for mammalian, bacterial, insect, yeast, and fungal expression may also be selected for this purpose.

The present invention also encompasses a cell line transfected with a recombinant plasmid containing the coding sequences of the antibodies or altered immunoglobulin molecules thereof. Host cells useful for the cloning and other manipulations of these cloning vectors are also conventional. However, most desirably, cells from various strains of *E*. *coli* are used for replication of the cloning vectors and other steps in the construction of altered antibodies of this invention.

Suitable host cells or cell lines for the expression of the antibody of the invention are preferably mammalian cells such as NS0, Sp2/0, CHO, COS, a fibroblast cell (e.g., 3T3), and myeloid cells, and more preferably a CHO or a myeloid cell. Human cells may be used, thus enabling the molecule to be modified with human glycosylation patterns. Alternatively, other eukaryotic cell lines may be employed. The selection of suitable mammalian host cells and methods for transformation, culture, amplification, screening and product production and purification are known in the art. See, e.g., *Sambrook et al*., cited above.

Bacterial cells may prove useful as host cells suitable for the expression of the recombinant Fabs of the present invention (see, e.g., Plückthun, A., Immunol. Rev., 130:151-188 (1992)). However, due to the tendency of proteins expressed in bacterial cells to be in an unfolded or improperly folded form or in a non-glycosylated form, any recombinant Fab produced in a bacterial cell would have to be screened for retention of antigen binding ability. If the molecule expressed by the bacterial cell was produced in a properly folded form, that bacterial cell would be a desirable host. For example, various strains of *E*. *coli* used for expression are well-known as host cells in the field of biotechnology. Various strains of *B. subtilis, Streptomyces,* other bacilli and the like may also be employed in this method.

Where desired, strains of yeast cells known to those skilled in the art are also available as host cells, as well as insect cells, e.g. *Drosophila* and *Lepidoptera* and viral expression systems. See, e.g. Miller *et al*., Genetic Engineering, 8:277-298, Plenum Press (1986) and references cited therein.

The general methods by which the vectors may be constructed, the transfection methods required to produce the host cells of the invention, and culture methods necessary to produce the altered antibody of the invention from such host cell are all conventional techniques. Likewise, once produced, the antibodies of the invention may be purified from the cell culture contents according to standard procedures of the art, including ammonium sulfate precipitation, affinity columns, column chromatography, gel electrophoresis and the like. Such techniques are within the skill of the art and do not limit this invention.

Yet another method of expression of the antibodies may utilize expression in a transgenic animal, such as described in U. S. Patent No. 4,873,316. This relates to an expression system using the animal's casein promoter which when transgenically incorporated into a mammal permits the female to produce the desired recombinant protein in its milk.

Once expressed by the desired method, the antibody is then examined for *in vitro* activity by use of an appropriate assay. Presently conventional ELISA assay formats are employed to assess qualitative and quantitative binding of the antibody to MAG. Additionally, other *in vitro* assays may also be used to verify neutralizing efficacy prior to subsequent human clinical studies performed to evaluate the persistence of the antibody in the body despite the usual clearance mechanisms.

The therapeutic agents of this invention may be administered as a prophylactic or post injury, or as otherwise needed. The dose and duration of treatment relates to the relative duration of the molecules of the present invention in the human circulation, and can be adjusted by one of skill in the art depending upon the condition being treated and the general health of the patient.

The mode of administration of the therapeutic agent of the invention may be any suitable route which delivers the agent to the host. The antagonists and antibodies, and pharmaceutical compositions of the invention are particularly useful for parenteral administration, i.e., subcutaneously, intramuscularly, intravenously, or intranasally.

Therapeutic agents of the invention may be prepared as pharmaceutical compositions containing an effective amount of the antagonist or antibody of the invention as an active ingredient in a pharmaceutically acceptable carrier. In the prophylactic agent of the invention, an aqueous suspension or solution containing the engineered antibody, preferably buffered at physiological pH, in a form ready for injection is preferred. The compositions for parenteral administration will commonly comprise a solution of the antagonist or antibody of the invention or a cocktail thereof dissolved in an pharmaceutically acceptable carrier, preferably an aqueous carrier. A variety of aqueous carriers may be employed, e.g., 0.9% saline, 0.3% glycine, and the like. These solutions are sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antagonist or antibody of the invention in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

Thus, a pharmaceutical composition of the invention for intramuscular injection could be prepared to contain 1 mL sterile buffered water, and between about 1 ng to about 100 mg, e.g. about 50 ng to about 30 mg or more preferably, about 5 mg to about 25 mg, of an antagonist or antibody of the invention. Similarly, a pharmaceutical composition of the invention for intravenous infusion could be made up to contain about 250 ml of sterile Ringer's solution, and about 1 to about 30 and preferably 5 mg to about 25 mg of an engineered antibody of the invention. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania.

It is preferred that the therapeutic agent of the invention, when in a pharmaceutical preparation, be present in unit dose forms. The appropriate therapeutically effective dose can be determined readily by those of skill in the art. To effectively treat stroke and other neurological diseases in a human, one dose of up to 700 mg per 70 kg body weight of an antagonist or antibody of this invention should be administered parenterally, preferably *i*.*v*. or i.m. (intramuscularly). Such dose may, if necessary, be repeated at appropriate time intervals selected as appropriate by a physician.

The antibodies described herein can be lyophilized for storage and reconstituted in a suitable carrier prior to use. This technique has been shown to be effective with conventional immunoglobulins and art-known lyophilization and reconstitution techniques can be employed.

In another aspect, the invention provides a pharmaceutical composition comprising anti-MAG antibody, and a pharmaceutically acceptable carrier for treatment or prophylaxis of stroke.

In a yet further aspect, the invention provides a pharmaceutical composition comprising anti-MAG antibody, including altered antibodies or a functional fragment thereof and a pharmaceutically acceptable carrier for inhibiting neurodegeneration and/or promoting functional recovery in a human patient suffering, or at risk of developing, a stroke.

The following examples illustrate the invention.

### Materials and Methods

### Anti-MAG monoclonal antibody

Anti-MAG monoclonal antibody was mouse anti-chick MAG antibody MAB 1567 obtained from Chemicon. The antibody has the following characteristics:
Antigen: myelin-associated glycoprotein (human, mouse, rat, bovine, chick, frog)
Isotype: IgG1
Neutralising ability: see DeBellard et al (1996) *Mol. Cell. Neurosci.* **7**, 89-101; Tang et al (1997) *Mol. Cell. Neurosci.* **9**, 333-346; Torigoe K and Lundborg G (1997) *Exp. Neurology* **150**, 254-262.

Control IgG1 mab was purchased from R+D Systems.

This antibody was sequenced and the results are shown in Figures 1 and 2. Figure 1 shows the sequence of the variable chain (SEQ ID No: 7) and constant chain (SEQ ID No: 8) of the light chain. Figure 2 shows the sequence of the variable chain (SEQ ID No 9) and constant chain (SEQ ID No 10) of the heavy chain.

### Intra-cerebral ventricular cannulation (for study 1 only)

Under halothane anaesthesia intra-cerebral ventricular (i.c.v.) cannulae were positioned in the left lateral cerebral ventricle (coordinates :1.6mm from the midline, 0.8mm caudal from bregma, 4.1mm from skull surface, incisor bar -3.2mm below zero according to Paxinos and Watson, 1986) All rats were singly housed to avoid damage to the guide or dummy cannula. 7 days following surgery, correct cannula placement was verified by an intense drinking response to Angiotensin II (100ng, Simpson, et al. 1978). Nine days later, animals underwent cerebral ischaemia.

### Transient Focal Cerebral Ischaemia

Transient (90 min) focal cerebral ischaemia was induced in male Sprague Dawley rats, each weighing between 300-350g. The animals were initially anaesthetised with a mixture of 5% halothane, 60% nitrous oxide and 30% oxygen, placed on a facemask and anaesthesia subsequently maintained at 1.5% halothane. Middle cerebral artery occlusion (MCAO) was carried out using the intraluminal thread technique as described previously (Zea Longa, et. al., 1989). Animals were maintained normothermic throughout the surgical procedure, allowed to recover for 1h in an incubator, before being singly housed. Only those animals with a neurological score of 3 1h post-occlusion were included in the study (as assessed using a 5-point scoring system: 0, no deficit; 1, contralateral reflex; 2, weakened grip; 3, circling; 4, immobile; 5, dead). Animals were maintained for up to 1 week at which time animals were killed by transcardial perfusion of 0.9% saline followed by 4% paraformaldehyde in 100mM phosphate buffer. The brains were post-fixed in 4% paraformaldehyde at 4°C for 48h at which time they were removed from the skulls and cut into 2mm blocks using a rat brain matrix. The 2mm sections were then paraffin embedded using a Shandon Citadel 1000 tissue processor, cut into 6µm sections using a microtome and mounted on poly-L-lysine coated slides. Sections were then processed for Cresyl Fast Violet (CFV) staining.

### Dosing regime

Anti-MAG monoclonal antibody and mouse IgG1 isotype control antibody were dialysed against sterile 0.9 % sodium chloride overnight and concentrated appropriately. Study 1 : Animals received 2.5µg of anti-MAGmab or 2.5µg mouse IgG i.c.v. 1, 24 and 72h following MCAO (5ul per dose).
Study 2 : Animals received 200µg of anti-MAG mab or 200µg mouse IgG i.v. 1 and 24 following MCAO.
Investigator was blinded to the identity of each dosing solution.

### Neurological assessment

Prior to induction of cerebral ischaemia, rats for Study 1 received training in beam walking and sticky label test. Animals not reaching criteria in both tests were excluded from further study. Following training, the remainder of the animals were stratified according to performance into two balanced groups. Throughout the neurological assessment, the investigators were blinded to the treatment group of the animal.

### Bilateral Sticky label test

The bilateral sticky label test (Schallert et al., Pharmacology Biochemistry and Behaviour 16 : 455-462, (1983)) was used to assess contralateral neglect/ipsilateral bias. This models tactile extinction observed in human stroke patients (Rose, et al. 1994). This test has been described in detail previously (Hunter, et al., Neuropharmacology 39 : 806-816 (2000); Virley et al Journal of Cerebral Blood Flow & Metabolism, 20 : 563-582 (2000)). Briefly, a round, sticky paper label was placed firmly around the hairless area of the forepaws with equal pressure with order of placement randomised (left, right). Training sessions were conducted for 6 days prior to MCAO, day 6 data was utilised as the pre-operative baseline (Day 0). Animals were given two trials 24 and 7d following MCAO, the data represents a mean of the two trials. The latency to contact and remove the labels were recorded and analysed using the logrank test (Cox, J. Royal Statistical Society B 34: 187-220 (1972)).

### Beam walking

Beam walking was used as a measure of hind-limb and fore-limb co-ordination by means of distance travelled across an elevated 100cm beam (2.3cm diameter, 48 cm off the floor) as previously described in detail (Virley et al Journal of Cerebral Blood Flow *&* Metabolism, 20 : 563-582 (2000)). Rats were trained to cross the beam from start to finish. For testing, each rat was given 2 trials 24 h and 7d following MCAO, the data represents a mean of the two trials. Statistical analysis was ANOVA followed by Student's t-test.

### The 27-point neurological score (Study-1)

This study consists of a battery of tests to assess neurological status including, paw placement, visual forepaw reaching, horizontal bar, contralateral rotation, inclined plane, righting reflex, contralateral reflex, motility & general condition, as described previously (Hunter, et al. Neuropharmacology 39: 806-816 (2000)) with the addition of grip strength measurements (scores 2 for good right fore-limb grip, 1 for weak grip). Total score = 27 for normal animal.

For study 2 this test was modified further: Grip strength - normal scores 3, good - 2, weak - 1, very weak - 0; Motility - normal scores 4, excellent - 3, very good - 2, good - 1, fair - 0; General Condition - normal scores 4, excellent - 3, very good - 2, good - 1, fair - 0; Circling - none scores 5, favours one-side scores 4, large circle - 3, medium circle - 2, small circle - 1, spinning - 0). Total score = 32 for a normal animal.

In both studies animals were tested 1, 24, 48h and 7d following MCAO, a healthy normal animal scores 27 or 32 respectively. Data are presented as median values, Statistical analysis was Kruskil Wallis ANOVA.

### Lesion assessment

Study 1 - For each animal, lesion areas were assessed in sections from three pre-determined levels in the brain (0, -2.0 and -6.0 mm from Bregma respectively). Neuronal damage was assessed using cresyl fast violet staining and the area of damage measured using an Optimas 6.1 imaging package. Data is expressed as mean area (mm²) ± sem. Study 2 - For each animal, lesion areas were assessed in sections from seven pre-determined levels in the brain (+3mm to -8mm w.r.t. Bregma). Neuronal damage was assessed using cresyl fast violet staining and the area of damage measured using an Optimas 6.1 imaging package. Data is expressed as mean area (mm²) ± sem.

### Results

### Study 1 - Intra-cerebral ventrical (i.c.v.) administration of anti-MAG mab

### Neurological score

One hour following MCAO animals in both treatment groups showed marked impairment in neurological score (median score 12 in each group). There was no significant difference between groups at this time. However, 24 (p=0.02), 48 (p=0.005) h and 7d (p=0.006) following MCAO animals treated with anti-MAG mab (2.5 µg, 1, 24 and 72h post-MCAO) showed significantly improved Total Neurological score compared to those treated with control IgG. Median neurological scores 24, 48h and 7d following MCAO in the IgG₁ treated group were 15, 14 and 18 respectively compared to 19.5, 21.5 and 22 in the anti-MAG mab treated animals. On further analysis of the individual behaviours comprising the total score, this significant improvement was mainly attributed to improved performance in the following tests : paw placement (24h, p=0.045; 48h, p=0.016; 7d, p=0.008), grip strength (24h, p=0.049 48h, p=0.0495; 7d, p=0.243), motility (24h, p=0.199; 48h, p=0.012; 7d, p=0.067), horizontal bar (24h, p=0.065; 48h, p=0.005; 7d, p=0.016), inclined plane (24h, p=0.006; 48h, p=0.006; 7d, p=0.169), visual forepaw reaching (48h, p=0.049, 7d, p=0.049) and the degree of circling (24h, p=0.417; 48h, p=0.034; 7d, p=0.183).

### Beam Walking

Prior to surgery all animals were trained to cross the beam (100cm). Twenty four hours following surgery there was a significant impairment on the distance travelled on the beam in both anti-MAG (50±18cm) and IgG₁ (22±14cm) treated animals compared to pre-operative values. Although not significant, anti-MAG treated animals showed marked improvement over IgG₁ treated animals in that they travelled twice the distance of IgG₁ treated animals 24h following tMCAO. Seven days following surgery however, while both groups showed marked improvement over time, the performance of animals treated with IgG remained significantly impaired compared to baseline (55±15cm; p=0.005). In contrast however 7d following MCAO, animals treated with anti-MAG mab (2.5µg 1, 24 and 72h, i.c.v post MCAO) performance was not significantly different from baseline (75±15cm; p=0.07). This data shows that anti-MAG mab treatment accelerated recovery of this beam walking task compared to mouse IgG₁ treated controls.

### Sticky label

Prior to surgery, animals in each of the treatment groups rapidly contacted and removed the labels from each forepaw, there was no significant difference in the groups prior to treatment (Table 1). Twenty-four hours and 7d following MCAO the latency to contact the left paw in each of the treatment groups remained relatively unaltered, while that of the right was markedly increased. However there was no significant differences between removal times in anti-MAG and IgG₁ treated animals. In addition 24h following MCAO, the latency to removal from both the left and right forepaw was significantly increased in both treatment groups compared to baseline, however in anti-MAG treated animals the latency to removal from the left paw was significantly shorter than that of IgG₁ treated animals (p=0.03). There was also a trend for reduced latency to removal from the right paw in anti-MAG treated animals compared to those treated with IgG₁ (p=0.08) (Table 1). At 7d there was some degree of recovery in IgG₁ treated animals in that the latency to removal times for each forepaw were reduced compared to those at 24h (Table 1). This data suggests that treatment of rats with anti-MAG mab accelerate the recovery in this sticky label test following tMCAO.

**Table 1 - Sticky label data**

| Day | Treatment | Contact Time (s) (Mean±sem) | | Removal Time (s) (Mean±sem) | |
|---|---|---|---|---|---|
| | | Left Forepaw | Right Forepaw | Left Forepaw | Right Forepaw |
| 0 | Anti-MAG | 2.4±0.2 | 3.6±0.5 | 12±2 | 12±2 |
| 0 | IgG₁ | 3.3±0.6 | 4.2±0.7 | 10±1 | 9±1 |
| 1 | Anti-MAG | 5.9±3.7 | 109.6±27.5 | *61±26 | 96±26 |
| 1 | IgG₁ | 3.6±0.5 | 71.8±31.7 | 130±21 | 156±19 |
| 7 | Anti-MAG | 3.8±1 | 36.4±10.2 | 54±23 | 80±30 |
| 7 | IgG₁ | 2.8±0.3 | 64±28 | 23±8 | 87±20 |

| | | | | | |
|---|---|---|---|---|---|
| (*p=0.03 Anti-MAG v's IgG₁ using the logrank test) | | | | | |

### Lesion area measurements

Administration of anti-MAG mab i.c.v, significantly reduced lesion area in two of the three brain levels examined compared to those animals treated with equal amounts of mouse IgG₁ when examined 7 days following tMCAO (Table 2).

**Table 2**

| | Mean Lesion Area ± sem (mm²) 7d following tMCAO | | |
|---|---|---|---|
| Treatment | 0 mm wrt Bregma | -2 mm wrt Bregma | -6 mm wrt Bregma |
| Anti-MAG mab (n=8) | *9±2 | ^{$}4±3 | ^{#}3 ± 1 |
| Mouse IgG₁ (n=9) | 14±1 | 12 ± 1 | 5 ± 1 |

| | | | |
|---|---|---|---|
| (*p=0.02, ^{$}p=0.03, ^{#}p=0.06, anti-MAG v's IgG₁, One-way, unpaired Students T-Test) | | | |

### Study 2 - Intra-venous (i.v.) administration

### Neurological score

One and 24 hours following MCAO animals in both groups showed marked impairment in neurological score. There was no significant difference between groups at this time, median scores 24h following anti-MAG mab and IgG₁ treatment were 20 and 18 respectively (p=0.5). Forty-eight hours following MCAO, animals treated with anti-MAG mab (200ug, i.v. 1 and 24h post-MCAO) showed significant improvement in paw placement (p=0.048) and grip strength (p=0.033). Seven days following the onset of cerebral ischaemia animals treated with anti-MAG mab continued to improve (paw placement p=0.041; grip strength, p=0.048; motility, p=0.05) and showed significant improvement in total neurological score (median score 25) compared to those treated with mouse IgG₁ (Median score 23, p=0.047).

### Lesion area measurements

The anti-MAG antibody when administered i.v. MCAO significantly reduced lesion area at 5 out of 7 pre-determined brain levels (+3 to -8 mm w.r.t. Bregma) compared to isotype controls, when examined 7d following MCAO.

| Brain level wrt Bregma | Mean lesion area ± SEM (mm²) - Anti- MAG treated | Mean lesion area ± SEM (mm²) - Mouse IgG₁ treated |
|---|---|---|
| 3 mm | *0.38 ± 0.27 | 1.77 ± 0.45 |
| 1 mm | *5.82 ± 1.65 | 9.627 ± 1.14 |
| -1 mm | 8.98 ± 2.58 | 12.07 ± 1.57 |
| -2 mm | 7.28 ± 1.92 | 10.04 ± 1.87 |
| -4 mm | *5.57 ± 1.06 | 10.38 ± 1.39 |
| -6mm | * 1.36 ± 0.51 | 4.43 ± 1.95 |
| -8 mm | *0.27 ± 0.27 | 1.93 ± 0.56 |

| | | |
|---|---|---|
| * p< 0.05 - Unpaired, one-way Students T-test | | |

### Conclusions

An anti-MAG monoclonal antibody administered either directly into the CSF or intravenously following transient middle-cerebral artery occlusion in the rat, both reduced the area of cell death and improved functional recovery compared to control treated animals. The degree of neuroprotection seen in these studies suggests that this effect can not be attributed to axonal sprouting as this would not result in neuronal sparing. The improvement in functional recovery seen 24 and 48h following MCAO probably reflects the degree of neuroprotection offered by this antibody compared to control treated animals. However, over time the animals appear to improve further, suggesting that blocking MAG activity can also enhance functional recovery over time.

The studies presented here provide evidence that blocking the actions of MAG provide both neuroprotection and enhanced functional recovery in a rat model of stroke, and therefore anti-MAG antibodies or MAG antagonists provide potential therapeutic agents for acute neuroprotection and/or the promotion of functional recovery following stroke. Anti-MAG antibodies or MAG antagonists also have potential use in the treatment of other neurological disorders where the degeneration of cells and or nerve fibres is apparent such as spinal cord injury, traumatic brain injury, peripheral neuropathy, Alzheimer's disease, fronto-temporal dementias (tauopathies), Parkinson's disease, Huntington's disease and Multiple Sclerosis.

### Example 2 - Chimeric antibody

Altered antibodies include chimeric antibodies which comprise variable regions deriving from one species linked to constant regions from another species. An example of a mouse-human chimeric anti-MAG antibody of the invention is provided in Figures 5 and 6. Mouse-human chimeras using human IgG1, IgG2, IgG3, IgG4, IgA, IgE, IgM or IgD constant regions may be produced, as may chimeras associating the mouse variable regions with heavy or light chain constant regions from non-human species.

Figure 5 provides the amino acid sequence of a chimeric immunoglobulin heavy chain in which the murine anti-MAG variable region is associated with a functional immunoglobulin secretion signal sequence, and with an altered form of the human IgG1 constant region, in which Kabat residues 248 and 250 have been mutated to alanine in order to disable the effector functions of binding to FcγRI and complement protein Clq (Duncan, A.R. and Winter, G. Localization of the Clq binding site on antibodies by surface scanning. Nature 332, 738-740, 1988. Duncan, A.R., Woolf, J.M., Partridge, L.J., Burton, D.R. and Winter, G. Localisation of the binding site for human FcR1 on IgG. Nature 332, 563-564, 1988). Such mutations are optionally made in order to customise the properties of an altered antibody to achieve a particular therapeutic effect - for example binding to and blocking the function of an antigen without activating lytic effector mechanisms. Other altered antibodies of the invention use human constant regions without such disabling mutations.

Figure 6 provides the amino acid sequence of a chimeric immunoglobulin light chain in which the murine anti-MAG variable region is associated with a functional immunoglobulin secretion signal sequence, and with the human kappa constant region.

From the information provided in Figures 5 and 6, cDNA inserts encoding these chimeric chains may be prepared by standard molecular biology techniques well known to those skilled in the art. Briefly, the genetic code is used to identify nucleotide codons encoding the desired amino acids, creating a virtual cDNA sequence encoding the chimeric protein. If the cDNA insert is desired to be expressed in a particular organism, then particularly favoured codons may be selected according to known codon usage biases. The desired nucleotide sequence is then synthesised by means of PCR amplification of a template comprising overlapping synthetic oligonucleotides which, as a contig, represent the desired sequence. The resulting product may also be modified by PCR or mutagenesis to attach restriction sites to facilitate cloning into a plasmid for expression or further manipulations.

## Claims

1. Use of a monoclonal IgG anti-MAG antibody in the manufacture of an intravenously administrable medicament for the treatment of ischemic stroke.

2. Use of claim 1 wherein the antibody is selected from the group consisting of; chimaeric, human, humanized.

3. Use of claim 1 or 2 for the treatment of ischemic stroke in humans.

4. Use of any one of claims 1 to 3 wherein the antibody is an IgG₁ antibody.

5. Use according to any preceding claim wherein the medicament comprises a pharmaceutically acceptable carrier.

## Patentansprüche

1. Verwendung eines monoklonalen IgG-Anti-MAG-Antikörpers in der Herstellung eines intravenös verabreichbaren Medikaments zur Behandlung von ischämischem Schlaganfall.

2. Verwendung gemäss Anspruch 1, worin der Antikörper aus der Gruppe ausgewählt ist, die aus chimären, humanen und humanisierten besteht.

3. Verwendung gemäss Anspruch 1 oder 2 zur Behandlung von ischämischem Schlaganfall bei Menschen.

4. Verwendung gemäss einem der Ansprüche 1 bis 3, worin der Antikörper ein IgG₁-Antikörper ist.

5. Verwendung gemäss einem der vorhergehenden Ansprüche, worin das Medikament einen pharmazeutisch akzeptablen Träger umfasst.

## Revendications

1. Utilisation d'un anticorps monoclonal IgG anti-MAG dans la production d'un médicament pouvant être administré par voie intraveineuse pour le traitement d'un ictus ischémique.

2. Utilisation suivant la revendication 1, dans laquelle l'anticorps est choisi dans le groupe consistant en : un anticorps chimère, un anticorps humain et anticorps humanisé.

3. Utilisation suivant la revendication 1 ou 2, pour le traitement d'un ictus ischémique chez l'homme.

4. Utilisation suivant l'une quelconque des revendications 1 à 3, dans laquelle l'anticorps est un anticorps IgG1.

5. Utilisation suivant l'une quelconque des revendications précédentes, dans laquelle le médicament comprend un support pharmaceutiquement acceptable.
